# EUROPEAN PATENT SPECIFICATION

(11) **EP 3 031 493 B1**
(45) Date of publication and mention of the grant of the patent: **06.06.2018**
(21) Application number: 15196078.8
(22) Date of filing: 24.11.2015
(51) Int. Cl.: A61N 1/40, A61N 5/02, A61N 5/04, H01Q 3/36, H01Q 3/46, H01Q 15/00, H01Q 21/06, H01Q 21/08

(54) **METAMATERIAL PHASED ARRAY FOR HYPERTHERMIA THERAPY**
METAMATERIALPHASENANORDNUNG FÜR HYPERTHERMIETHERAPIE
RÉSEAU PHASÉ À MÉTA-MATÉRIAU POUR THÉRAPIE À HYPERTHERMIE

(30) Priority: 11.12.2014 US 201414567604
(43) Date of publication of application: 15.06.2016
(73) Proprietor: Palo Alto Research Center Incorporated, Palo Alto, CA 94304 (US)
(72) Inventor: CASSE, Bernard D., Saratoga, CA 95070 (US); LIU, Victor, Mountain View, CA 94040 (US); VOLKEL, Armin R., Mountain View, CA 94040 (US); DANIEL, George, Palo Alto, CA 94306 (US)
(74) Representative: Gill Jennings & Every LLP

(56) References cited:
- EP-A1- 2 975 693
- EP-A1- 2 975 694
- CN-A- 102 784 436
- US-A1- 2008 140 063
- US-A1- 2008 284 674
- US-A1- 2011 199 273

## Description

### TECHNICAL FIELD

This disclosure relates generally to hyperthermia therapy systems and methods pertaining to such systems.

### BACKGROUND

Hyperthermia therapy is a type of medical treatment in which body tissue is exposed to slightly higher temperatures (e.g. greater than about 100 F) to damage and kill cancer cells. Hyperthermia therapy helps make cancer cells more vulnerable to the effects of other treatments, like radiation therapy and certain chemotherapy drugs. Hyperthermia therapy is a promising treatment option for patients with advanced or recurrent cancer.

EP 2975693 A1 discloses a metamaterial-based phase shifting element and phased array. EP 2975694 A1 discloses a metamaterial-based object-detection system. CN 102784436 A discloses a microwave hyperthermia therapy device. US 2008/284674 A1 discloses a digital control architecture for a tunable impedance surface.

### SUMMARY

The present invention provides a device according to claim 1 and a method according to claim 10. Preferred embodiments are defined in the depended claims. Aspects, embodiments or examples of the present disclosure which do not fall within the scope of the appended claims are not part of the present invention.

Some embodiments are directed to a device having a phase shifting element array including a plurality of metamaterial structures that resonate in response to an input electromagnetic (EM) signal. The phase shifting element array generates an output EM signal that is a sum of component output electromagnetic signals generated respectively by the metamaterial structures and is configured to propagate wirelessly through at least a portion of a patient's body. The device includes a control circuit configured to control one or both of phases and amplitudes of the component electromagnetic output signals so that at least one of constructive and destructive interference between the component output electromagnetic signals causes the output signal to have a higher intensity EM radiation at a target region interior to the body and to have a zero or low intensity radiation at a non-target region interior to the body, and so that the output signal is caused to scan an area of a patient's body into which the output signal is directed, and is configured to generate scan direction data that includes the instantaneous scan direction of the output signal; a receiver circuit configured to detect a portion of the output signal that is reflected from one or more structures in the scan area interior to the body; and a signal processing circuit configured to combine information from the scan direction and the detected portion of the output signal to provide image information related to a body structure that might be present in the area. The higher intensity EM radiation generates a tissue temperature suitable for hyperthermia therapy at the target region. For example, the tissue temperature at the target region may be in in range of about 40 C to 50 C. The control circuit can be configured to control at least one of position, focus, and intensity of the higher intensity EM radiation at the target region.

According to some aspects, the control circuit includes variable capacitors electrically coupled respectively to the metamaterial structures so that a change in capacitance of one of the variable capacitors changes a phase of a component output signal of an associated metamaterial structure. The control circuit includes a signal generator configured to generate control signals that control capacitances of the variable capacitors. The input EM signal may propagate to the array of metamaterial structures through a wire probe antenna and/or through a waveguide.

According to some aspects, each metamaterial structure includes a first metal layer structure, an electrically isolated second metal layer structure, and a dielectric layer disposed between the first and second metal structures. The first and second metal layer structures are cooperatively configured such that the metamaterial structure resonates at a frequency of the input EM signal at a fixed capacitance. The first metal layer structure may be disposed on an upper dielectric surface of the dielectric layer. The metamaterial structure may further include a third metal layer structure disposed on the upper dielectric surface and spaced apart from the first metal layer structure. A variable capacitor has a first terminal electrically coupled to the first metal layer structure and a second terminal electrically coupled to the third metal layer structure.

In some embodiments, the first metal layer structure is disposed on an upper dielectric surface of the dielectric layer. The metamaterial structure further comprises a third metal layer structure disposed on the upper dielectric surface and spaced apart from the first metal layer structure. A second metamaterial structure comprises a fourth metal layer structure disposed on a lower dielectric surface and is spaced apart from the second metal layer structure. A second variable capacitor has a first terminal electrically coupled to the second metal layer structure and a second terminal electrically coupled to the fourth metal layer structure. For example, the metamaterial structure on the upper dielectric surface may be a mirror image of the metamaterial structure on the lower dielectric surface.

According to some embodiments, the first metal layer structure comprises a patterned planar structure defining one or more open regions. For example, the first metal layer structure can include a peripheral frame portion including an outer peripheral edge, one or more radial arms, each radial arm having a first end integrally connected to the peripheral frame portion and extending inward from the peripheral frame portion toward a central region of the metamaterial structure, and an inner structure integrally connected to second ends of the one or more radial arms, the inner structure being spaced from the peripheral frame portion.

In some configurations, the control signal is configured to control the component EM output signals to scan the output signal across a detection area. The control circuit may be configured to generate beam direction data indicating instantaneous scan direction of the output signal. The device can include a detector circuit configured to detect a portion of the output signal reflected from a structure interior to the body and a signal processing circuit configured to combine the scan direction and the reflected portion of the output signal and to provide information about the structure. For example, the information may comprise one or more of presence, size, location, and image information.

Some embodiments are directed to a method. An output EM signal is generated that is a sum of component output electromagnetic signals generated respectively by a plurality of metamaterial structures that resonate in response to an input electromagnetic (EM) signal. The output EM is propagated wirelessly through at least a portion of a patient's body. One or both of phases and amplitudes of the component electromagnetic output signals are controlled so that at least one of constructive and destructive interference between the component output electromagnetic signals causes the output signal to scan an area of a patient's body into which the output signal is directed, generating scan direction data that includes the instantaneous scan direction of the output signal; detecting a portion of the output signal that is reflected from one or more structures in the scan area interior to the body; combining information from the scan direction and the detected portion of the output signal to provide image information related to a body structure that might be present in the area. The higher intensity electromagnetic radiation generates a tissue temperature suitable for hyperthermia therapy at the target region.

Beam direction data is generated that indicates instantaneous scan direction of the output signal. A portion of the output signal reflected from a structure interior to the body is detected. The scan direction and the reflected portion of the output signal are combined to provide information about the structure.

### BRIEF DESCRIPTION OF THE DRAWINGS

FIG. 1A is a block diagram of a system in accordance with some embodiments;
FIG. 1B shows a therapy system in accordance with various embodiments that is in use;
FIG. 1C is a simplified side view showing a phase shifting apparatus according to a generalized embodiment of the present invention;
FIG. 2 is a diagram showing exemplary phase shifting characteristics associated with operation of the phase shifting apparatus of FIG. 1;
FIGS. 3A and 3B are exploded perspective and assembled perspective views, respectively, showing a phase shifting element according to an exemplary embodiment;
FIG. 3C is an exploded and assembled perspective view showing a phase shifting element that includes first and second metamaterial structures according to an exemplary embodiment;
FIG. 4 is a cross-sectional side view showing a phase shifting apparatus including the phase shifting element of FIG. 3B according to another exemplary embodiment;
FIG. 5 is a perspective view showing a phase shifting element including an exemplary patterned metamaterial structure according to another embodiment;
FIG. 6 is a cross-sectional side view showing a simplified phased array system including four phase shifting elements according to another exemplary embodiment;
FIG. 7 is a simplified perspective view showing a phase shifting element array according to another exemplary embodiment;
FIG. 8 is a simplified diagram depicting a phased array system including the phase shifting element array of FIG. 7 according to another embodiment;
FIG. 9 is simplified diagram showing a phased array system including metamaterial structures disposed in a two-dimensional pattern according to another exemplary embodiment; and
FIGS. 10A, 10B and 10C are diagrams depicting emitted beams generated in various exemplary directions by the phased array system of FIG. 9.
FIGS. 10D, 10E, 10F, and 10G provide modeling data that demonstrates focusing the local hyperthermia beam in x, y, and depth (into the body) dimensions;
FIG. 11 is a block diagram of an imaging system in accordance with some embodiments; and
FIG. 12 is a simplified side view showing an imaging system according to some embodiments.

The figures are not necessarily to scale. Like numbers used in the figures refer to like components. However, it will be understood that the use of a number to refer to a component in a given figure is not intended to limit the component in another figure labeled with the same number.

### DETAILED DESCRIPTION

Embodiments described herein involve a treatment and/or imaging system useful for tumor treatment and/or detection. The system is based on a phase shifting array that selectively provides electromagnetic radiation to a target region within the patient's body. The phase shifting array includes a plurality of metamaterial structures that resonate in response to an input electromagnetic (EM) signal. The phase shifting element array generates an output EM signal that is the combination of component output electromagnetic signals generated respectively by each of the metamaterial structures. The output signal is configured to propagate wirelessly through at least a portion of a patient's body. A control circuit controls one or both of phases and amplitudes of the component electromagnetic output signals so that at least one of constructive and destructive interference between the component output electromagnetic signals causes the output signal to have a higher intensity EM radiation at a target region interior to the body and to have a zero or low intensity radiation at a non-target region interior to the body. The higher intensity EM radiation generates a specified amount of heat flux the target region, thus providing noninvasive hyperthermia treatment within the patient's body.

According to some aspects, the system may be configured to control the phases of the respective component output signals so that the output signal can be caused to scan or "sweep" an area or region into which the output signal is directed. The control circuit generates scan direction data that includes the instantaneous scan direction of the output signal. The system includes a receiver circuit configured to detect a portion of the output signal that is reflected from one or more structures interior to the body. Signal processing circuit combines the scan direction and the detected portion of the output signal to provide information about presence and location of the structures.

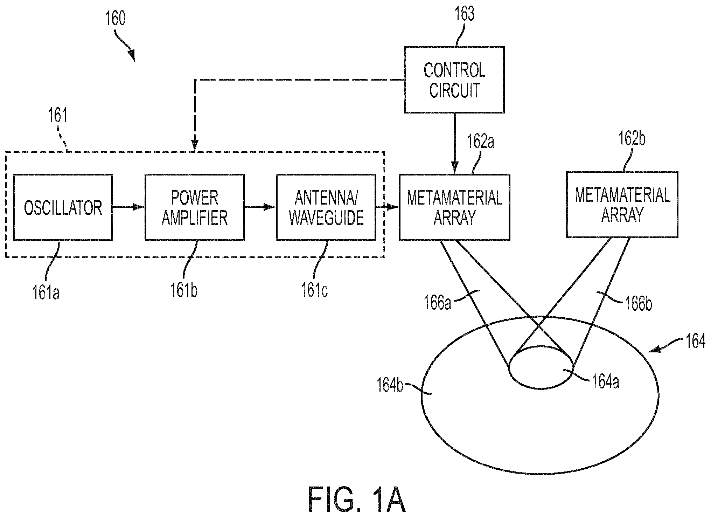

FIG. 1A is a schematic diagram of a system 160 in accordance with some embodiments. FIG. 1A depicts an input source 161 that provides an input electromagnetic (EM) radiation signal to a phase shifting array 162. A control circuit 163 is coupled to control the phase shifting array 162 and is optionally coupled to control the input source. According to various implementations, the control circuit 163 may be capable of controlling one or more of the phase of each component output signal, the amplitude of each component output signal, the frequency of the input signal and the amplitude of the input signal. The input source 161 includes a signal generator 161a such as an oscillator, coupled to a power amplifier 161b that boosts the input signal. According to some implementations, the input source 161 may produce a continuous wave (CW) voltage oscillating sinusoidally at a specified microwave frequency, e.g., 3 kHz to 300 GHz or in some implementations about 915 MHz. The input signal can be transmitted to the phase shifting array through an antenna and/or waveguide 161c, e.g., a wire probe antenna disposed within a metal waveguide structure that causes the input signal to be incident on the phased array as described in more detail below. The phased array 162 is configured to provide beam shaping and beam forming of the output signal beam due to constructive and/or destructive interference between the component output electromagnetic signals. The beam forming/beam shaping causes the output beam 166a to have a higher intensity EM radiation at a target region 164a interior to the patient's body 164 and to have a zero or low intensity radiation at a non-target region 164b interior to the patient's body 164. The higher intensity EM radiation can be controlled to a specified amount of heat flux the target region 164a to provide noninvasive hyperthermia treatment within the patient's body.

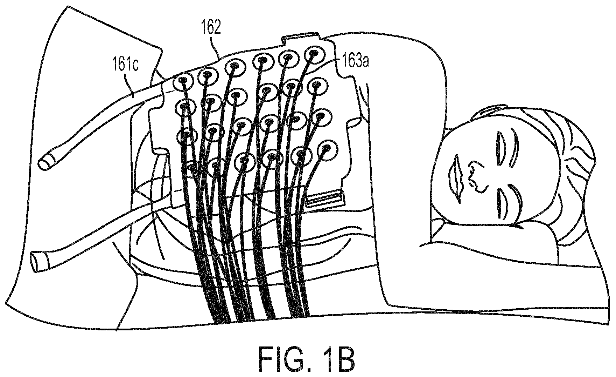

In some scenarios, multiple phased arrays 162a, 162b may be used to produce multiple output beams 166a, 166b. Optionally, in some embodiments, a first output signal beam 166a may be substantially orthogonal to a second output signal beam 166b. FIG. 1B shows a phased array 162 positioned relative to a patient with control lines 163a and waveguides 161c visible.

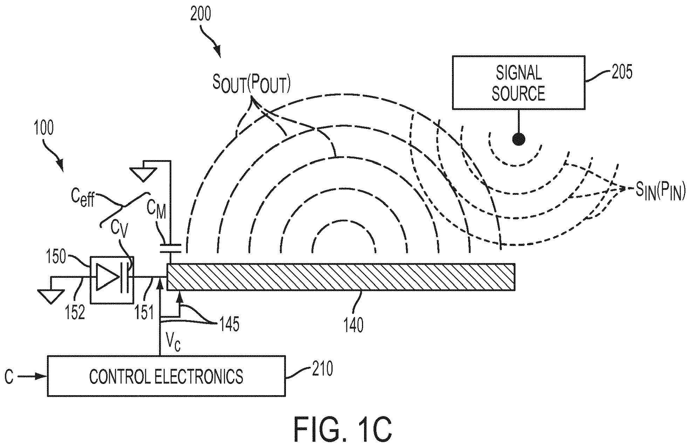

FIG. 1C is a simplified side view showing a system 200 including at least one metamaterial-based phase shifting element 100 according to a generalized exemplary embodiment. Phase shifting element 100 utilizes a metamaterial structure 140 to produce an output signal S_{OUT} having the same radio wave frequency as that of an applied/received input signal S_{IN}, and utilizes a variable capacitor 150 to control a phase p_{OUT} of output signal S_{OUT} by way of an applied phase control signal (e.g., either an externally supplied digital signal C or a direct-current control voltage Vc). System 200 also includes a portion of a signal source 205 (e.g., a feed horn or a leaky-wave feed) disposed in close proximity to phase shifting element 100 and configured to generate input signal S_{IN} at a particular radio wave frequency (e.g., in the range of 3 kHz to 300 GHz) and an input phase p_{IN}, where the radio wave frequency matches resonance characteristics of phase shifting element 100, and a control circuit 210 (e.g., including a digital-to-analog converter (DAC) that is controlled by any of a field programmable gate array (FPGA), an application specific integrated circuit (ASIC, or a micro-processor) that is configured to generate phase control voltages Vc applied to variable capacitor 150 at voltage levels determined in accordance with (e.g., directly or indirectly proportional to) a preprogrammed signal generation scheme or an externally supplied phase control signal C.

Metamaterial structure 140 is preferably a layered metal-dielectric composite architecture, but may be engineered in a different form, provided the resulting structure is configured to resonate at the radio frequency of applied input signal S_{IN}, and has a large phase swing near resonance such that metamaterial structure 140 generates output signal S_{OUT} at the input signal frequency by retransmitting (e.g., reflecting/scattering) input signal S_{IN}. In providing this resonance, metamaterial structure 140 is produced with an inherent "fixed" capacitance C_{M} and an associated inductance that collectively provide the desired resonance characteristics. As understood in the art, the term "metamaterial" identifies an artificially engineered structure formed by two or more materials and multiple elements that collectively generate desired electromagnetic properties, where metamaterial achieves the desired properties not from its composition, but from the exactingly-designed configuration (e.g., the precise shape, geometry, size, orientation and arrangement) of the structural elements formed by the materials. As used herein, the phrase "metamaterial structure" is intended to mean a dynamically reconfigurable/tunable metamaterial having radio frequency resonance and large phase swing properties suitable for the purpose set forth herein. The resulting structure affects radio frequency (electromagnetic radiation) waves in an unconventional manner, creating material properties which are unachievable with conventional materials. Metamaterial structures achieve their desired effects by incorporating structural elements of sub-wavelength sizes (having a period of λ/2 or less), e.g. features that are actually smaller than the radio frequency wavelength of the waves they affect. In the practical embodiments described below, metamaterial structure 140 is constructed using inexpensive metal film or PCB fabrication technology that is tailored by solving Maxwell's equations to resonate at the radio frequency of applied input signal S_{IN}, whereby the metamaterial structure 140 generates output signal S_{OUT} at the input signal frequency by retransmitting (e.g., reflecting/scattering) the input signal S_{IN}.

Variable capacitor 150 is connected between metamaterial structure 140 and ground (or other fixed direct-current (DC) voltage supply). As understood in the art, variable capacitors are typically two-terminal electronic devices configured to produce a capacitance that is intentionally and repeatedly changeable by way of an applied electronic control signal. In this case, variable capacitor 150 is coupled to metamaterial structure 140 such that an effective capacitance C_{eff} of metamaterial structure 140 is determined by a product of inherent capacitance C_{M} and a variable capacitance Cv supplied by variable capacitor 150. The output phase of metamaterial structure 140 is determined in part by effective capacitance C_{eff}, so output phase p_{OUT} of output signal S_{OUT} is "tunable" (adjustably controllable) to a desired phase value by way of changing variable capacitance Cv, and this is achieved by way of changing the phase control signal (e.g., digital control signal C and/or DC bias voltage Vc) applied to variable capacitor 150.

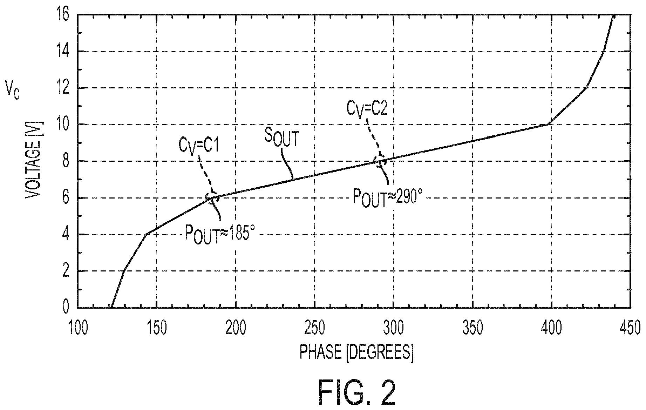

FIG. 2 is a diagram showing exemplary phase shifting characteristics associated with operation of phase shifting apparatus 200. In particular, FIG. 2 shows how output phase p_{OUT} of output signal S_{OUT} changes in relation to phase control voltage Vc. Because output phase p_{OUT} varies in accordance with effective capacitance C_{eff} of metamaterial structure 140 which in turn varies in accordance with variable capacitance Cv generated by variable capacitor 150 on metamaterial structure 140 (shown in FIG. 1C), FIG. 2 also effectively depicts operating characteristics of variable capacitor 150 (e.g., FIG. 2 effectively illustrates that variable capacitance Cv varies in accordance with phase control voltage Vc by way of showing how output phase p_{OUT} varies in accordance with phase control voltage Vc). For example, when phase control voltage Vc has a voltage level of 6V, variable capacitor 150 generates variable capacitance Cv at a corresponding capacitance level (indicated as "Cv=C1") and metamaterial structure 140 generates output signal S_{OUT} at an associated output phase p_{OUT} of approximately 185°. When phase control voltage Vc is subsequently increased from 6V to a second voltage level (e.g., 8V), variable capacitor 150 generates variable capacitance at a second capacitance level (indicated as "Cv=C2") such that metamaterial structure 140 generates output signal S_{OUT} at an associated second output phase p_{OUT} of approximately 290°.

Referring again to FIG. 1C, phase control voltage Vc is applied across variable capacitor 150 by way of a conductive structure 145 that is connected either to metamaterial structure 140 or directly to a terminal of variable capacitor 150. Specifically, variable capacitor 150 includes a first terminal 151 connected to metamaterial structure 140 and a second terminal 152 connected to ground. As indicated in FIG. 1C, conductive structure 145 is either connected to metamaterial structure 140 or to first terminal 151 of variable capacitor 150 such that, when phase control voltage Vc is applied to conductive structure 145, variable capacitor 150 generates an associated variable capacitance Cv having a capacitance level that varies in accordance with the voltage level of phase control voltage Vc in the manner illustrated in FIG. 2 (e.g., the capacitance level of variable capacitance Cv changes in direct proportion to phase control voltage Vc).

As set forth in the preceding exemplary embodiment, some scenarios involve a phase shifting methodology involving control over radio wave output signal phase p_{OUT} by selectively adjusting effective capacitance C_{eff} of metamaterial structure 140, which is implemented in the exemplary embodiment by way of controlling variable capacitor 150 using phase control voltage Vc to generate and apply variable capacitance Cv onto metamaterial structure 140. Although the use of variable capacitor 150 represents the presently preferred embodiment for generating variable capacitance Cv, those skilled in the art will recognize that other circuits may be utilized to generate a variable capacitance that controls effective capacitance C_{eff} of metamaterial structure 140 in a manner similar to that described herein. Accordingly, the novel methodology is alternatively described as including: causing metamaterial structure 140 to resonate at the radio wave frequency of input signal S_{IN}; applying a variable capacitance Cv (e.g., from any suitable variable capacitance source circuit) to metamaterial structure 140 such that effective capacitance C_{eff} of metamaterial structure 140 is altered by variable capacitance Cv; and adjusting variable capacitance Cv (e.g., by way of controlling the suitable variable capacitance source circuit) until effective capacitance C_{eff} of metamaterial structure 140 has a capacitance value that causes metamaterial structure 140 to generate radio frequency output signal S_{OUT} with output phase p_{OUT} set at a desired phase value (e.g., 290°).

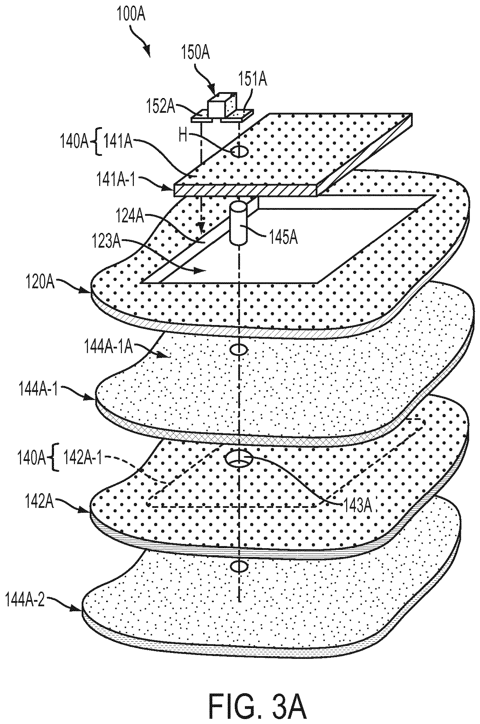

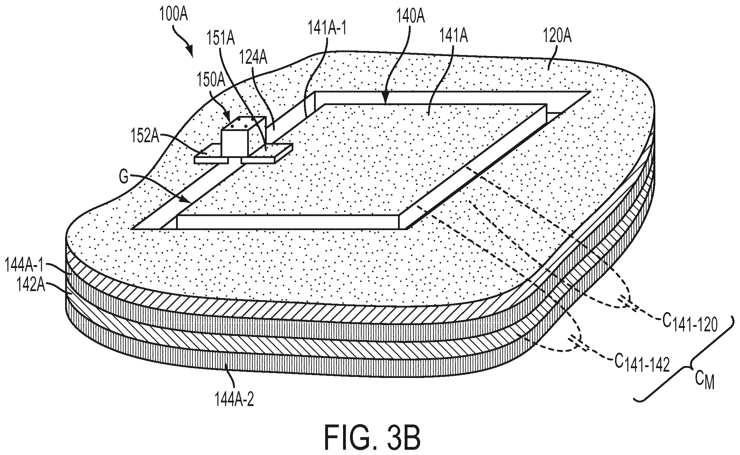

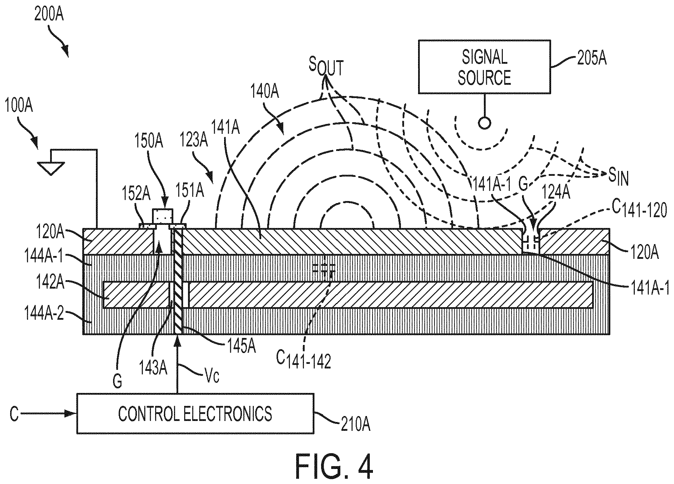

As mentioned above, some implementations involve the use of layered metamaterial structures. FIGS. 3A and 3B are exploded perspective and assembled perspective views, respectively, showing a phase shifting element 100A including a two-terminal variable capacitor 150A and a metamaterial structure 140A having an exemplary three-level embodiment. FIG. 4 shows a phase shifting apparatus 200A including phase shifting element 100A in cross-sectional side view. Beneficial features and aspects of the three-layer structure used to form metamaterial structure 140A, and their usefulness in forming metamaterial-based phase shifting element 100A and apparatus 200A, are described below with reference to FIGS. 3A, 3B and 4.

Referring to FIGS. 3A and 3B three-layer metamaterial structure 140A is formed by an upper/first metal layer (island) structure 141A, an electrically isolated (e.g., floating) backplane (lower/second metal) layer structure 142A, and a dielectric layer 144A-1 sandwiched between upper island structure 141A and backplane layer 142A, where island structure 141A and backplane layer 142A are cooperatively tailored (e.g., sized, shaped and spaced by way of dielectric layer 144A-1) such that the composite three-layer structure of metamaterial structure 140A has an inherent (fixed) capacitance C_{M} that is at least partially formed by capacitance C₁₄₁₋₁₄₂ (e.g., the capacitance between island structure 141A and backplane layer 142A), and such that metamaterial structure 140A resonates at a predetermined radio wave frequency (e.g., 2.4GHz). As discussed above, an effective capacitance of metamaterial structure 140A is generated as a combination of fixed capacitance C_{M} and an applied variable capacitance, which in this case is applied to island structure 141A by way of variable capacitor 150A. In this arrangement, island structure 141A acts as a wavefront reshaper, which ensures that the output signal S_{OUT} is directed in the upward direction only (e.g., such that the radio frequency output signal is emitted from island structure 141A in a direction away from backplane layer 142A), and which minimizes power consumption because of efficient scattering with phase shift.

According to a presently preferred embodiment, dielectric layer 144A-1 comprises a lossless dielectric material. For example, in some implementations, the dielectric material can comprise RT/duroid® 6202 Laminates, Polytetrafluoroethylene (PTFE), and TMM4® dielectric, all produced by Rogers Corporation of Rogers, CT. The use of such lossless dielectric materials mitigates absorption of incident radiation (e.g., input signal S_{IN}), and ensures that most of the incident radiation energy is re-emitted in output signal S_{OUT}. An optional lower dielectric layer 144A-2 is provided to further isolate backplane layer 142A, and to facilitate the backside mounting of control circuits in the manner described below.

According to another feature, both island (first metal layer) structure 141A and a base (third) metal layer structure 120A are disposed on an upper surface 144A-1A of dielectric layer 141A-1, where base metal structure 120A is spaced from (e.g., electrically separated by way of a gap G) island structure 141A. Metal layer structure 120A is connected to a ground potential during operation, base, whereby base layer structure 120A facilitates low-cost mounting of variable capacitor 150A during manufacturing. For example, using pick-and-place techniques, variable capacitor 150A is mounted such that first terminal 151A is connected (e.g., by way of solder or solderless connection techniques) to island structure 141A, and such that second terminal 152A is similarly connected to base metal structure 120A.

According to some implementations, base metal structure 120A comprises a metal film or PCB fabrication layer that entirely covers upper dielectric surface 144A-1A except for the region defined by an opening 123A, which is disposed inside an inner peripheral edge 124A, where island structure 141A is disposed inside opening 123A such that an outer peripheral edge 141A-1 of is structure 141A is separated from inner peripheral edge 124A by peripheral gap G, which has a fixed gap distance around the entire periphery. By providing base metal structure 120A such that it substantially covers all portions of upper dielectric surface 144A-1A not occupied by island structure 141A, base metal layer 120A forms a scattering surface that supports collective mode oscillations, and ensures scattering of the wave in the forward direction. In addition, island structure 141A, backplane layer 142A and base metal structure 120A are cooperatively configured (e.g., sized, shaped and spaced) such that inherent (fixed) capacitance C_{M} includes both the island-backplane component C₁₄₁₋₁₄₂ and an island-base component C₁₄₁₋₁₂₀, and such that metamaterial structure 140A resonates at the desired radio wave frequency. In this way, base metal layer 120A provides the further purpose of effectively forming part of metamaterial structure 140A by enhancing fixed capacitance C_{M}.

According to another feature, both base (third) metal layer structure 120A and island (first metal layer) structure 141A comprise a single metal (e.g., both base metal structure 120A and island structure 141A comprise the same, identical metal composition, e.g., copper). This single-metal feature facilitates the use of low-cost manufacturing techniques in which a single metal film or PCB fabrication is deposited on upper dielectric layer 144A-1A, and then etched to define peripheral gap G. In other embodiments, different metals may be patterned to form the different structures.

According to another feature shown in FIG. 3A, a metal via structure 145A is formed using conventional techniques such that it extends through lower dielectric layer 144A-2, through an opening 143A defined in backplane layer 142A, through upper dielectric layer 144A-1, and through an optional hole H formed in island structure 141A to contact first terminal 151A of variable capacitor 150A. This via structure approach facilitates applying phase control voltages to variable capacitor 150A without significantly affecting the electrical characteristics of metamaterial structure 140A. As set forth below, this approach also simplifies the task of distributing multiple control signals to multiple metamaterial structures forming a phased array.

FIG. 4 is a cross-sectional side view showing a phase shifting apparatus 200A generating output signal S_{OUT} at an output phase p_{OUT} determined an externally-supplied phase control signal C. Apparatus 200A includes a signal source 205A, phase shifting element 100A, and a control circuit 210A. Signal source 205A includes a suitable signal generator (e.g., a feed horn) that generates an input signal S_{IN} at a specific radio wave frequency (e.g., 2.4GHz), and is positioned such that input signal S_{IN} is directed onto phase shifting element 100A, which is constructed as described above to resonate at the specific radio wave frequency (e.g., 2.4GHz) such that it generates an output signal S_{OUT}. Control circuit 210A is configured to generate a phase control voltage Vc in response to phase control signal C such that phase control voltage Vc changes in response to changes in phase control signal C. Phase control voltage Vc is transmitted to variable capacitor 150A, causing variable capacitor 150A to generate and apply a corresponding variable capacitance onto island structure 141A, whereby metamaterial structure 140A is caused to generate output signal S_{OUT} at an output phase p_{OUT} determined by phase control signal C. Note that control circuit 210A is mounted below dielectric layer 144A-2 (e.g., below backplane layer 142A), and phase control voltage Vc is transmitted by way of conductive via structure via 145A to terminal 151A of variable capacitor 150A.

Those skilled in the art understand that the metamaterial structures generally described herein can take many forms and shapes, provided the resulting structure resonates at a required radio wave frequency, and has a large phase swing near resonance. The embodiment shown in FIGS. 3A, 3B and 4 utilizes a simplified square-shaped metamaterial structure and a solid island structure 141A to illustrate basic concepts according to some embodiments. Specifically, metamaterial structure 140A is formed such that inner peripheral edge 124A surrounding opening 123A in base metal structure 120A and outer peripheral edge 141A-1 of island structure 141A comprise concentric square shapes such that a width of peripheral gap G remains substantially constant around the entire perimeter of island structure 141A. The use of square-shaped structures may simplify the geometric construction and provide limited degrees of freedom that simplify the mathematics needed to correlate phase control voltage Vc with desired capacitance change and associated phase shift. In alternative embodiments, metamaterial structures are formed using shapes other than squares (e.g., round, triangular, rectangular/oblong).

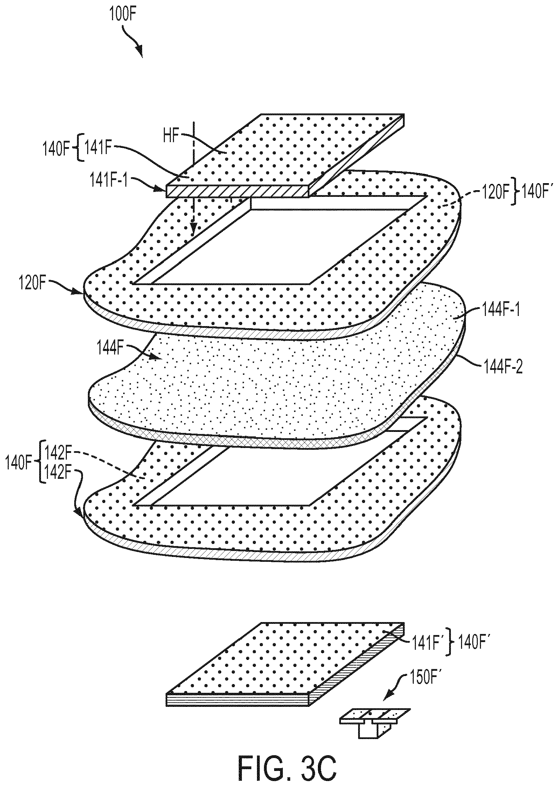

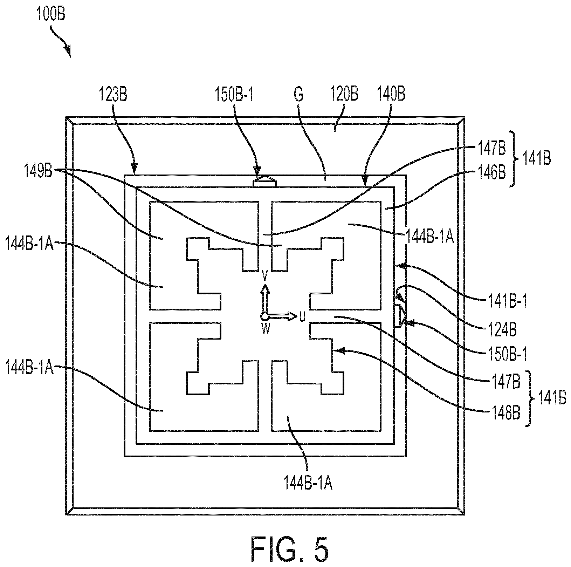

FIG. 3C shows a phase shifting element 100F that includes a first metamaterial structure 140F, a second metamaterial structure 140F', and a two-terminal variable capacitor 150F'. In some embodiments, the second metamaterial structure 140F' is a mirror image of the first metamaterial structure 140F. The metal structure at the top of FIG. 3C (elements 120F and 141F) is the same or similar to the metal structure at the top of FIG. 3C (elements 142F and 141F'). The first and second metamaterial structures may comprise patterned layers, e.g., as depicted in FIG. 5.

First metamaterial structure 140F is formed by an upper/first metal layer (island) structure 141F, a second metal layer structure 142F, and a dielectric layer 144F sandwiched between the first metal layer (island) structure 141F and second metal layer 142F.

Second metamaterial structure 140F' is formed by an lower/fourth metal layer (island) structure 141F', a third metal layer structure 120F, and the dielectric layer 144F sandwiched between the fourth metal layer (island) structure 141F' and third metal layer structure 120F. The first and second metamaterial structures are cooperatively tailored (e.g., sized, shaped and spaced by way of dielectric layer 144F) such that the metamaterial structures 140F, 140F' have an inherent (fixed) capacitance that enables the first and second metamaterial structures 140F, 140F' to resonate at a predetermined radio wave frequency. An effective capacitance of metamaterial structures 140F, 140F' is generated as a combination of fixed capacitances and applied variable capacitances, which in this case is applied to island structure 141F and/or 141F' by way of variable capacitor 150F'.

Both the first metal layer (island) structure 141F and the third metal layer structure 120F are disposed on an upper surface 144F-1 of dielectric layer 144F, where the third metal layer structure 120F is spaced from (e.g., electrically separated by way of a gap) first metal layer (island) structure 141F. Third metal layer structure 120F may be connected to a ground potential during operation.

Both the fourth metal layer (island) structure 141F' and a second metal layer structure 142F are disposed on a lower surface 144F-2 of dielectric layer 144F, where second metal layer structure 142F is spaced from (e.g., electrically separated by way of a gap) fourth metal layer (island) structure 141F'. Second metal layer structure 142F may be connected to a ground potential during operation. Variable capacitor 150F' is mounted such that the first terminal of variable capacitor 150F' is connected (e.g., by way of solder or solderless connection techniques) to fourth metal layer (island) structure 141F', and the second terminal of variable capacitor 150F' is similarly connected to the second metal layer structure 142F.

FIG. 5 is a top view showing a phase shifting element 100B including an exemplary patterned metamaterial structure 140B according to an exemplary specific embodiment. In this embodiment, island structure 141B is formed as a patterned planar structure that defines open regions 149B (e.g., such that portions of upper dielectric surface 144B-1A are exposed through the open regions). In this example, island structure 141B includes a square-shaped peripheral frame portion 146B including an outer peripheral edge 141B-1 that is separated by a peripheral gap G from an inner peripheral edge 124B of base metal layer portion 120B, which is formed as described above, four radial arms 147B having outer ends integrally connected to peripheral frame portion 146B and extending inward from frame portion 146B, and an inner (in this case, "X-shaped") structure 148B that is connected to inner ends of radial arms 147B. Structure 148B extends into open regions 149B, which are formed between radial arms 147B and peripheral frame 146B. Metamaterial structure 140B is otherwise understood to be constructed using the three-layer approach described above with reference to FIGS. 3A, 3B and 4. Although the use of patterned metamaterial structures may complicate the mathematics associated with correlating control voltage and phase shift values, the X-shaped pattern utilized by metamaterial structure 140B is presently believed to produce more degrees of freedom than is possible using solid island structures, leading to close to 360° phase swings, which in turn enables advanced functions such as beam steering at large angles (e.g., greater than plus or minus 60°). In addition, although metamaterial structure 140B is shown as having a square-shaped outer peripheral edge, patterned metamaterial structures having other peripheral shapes may also be beneficially utilized.

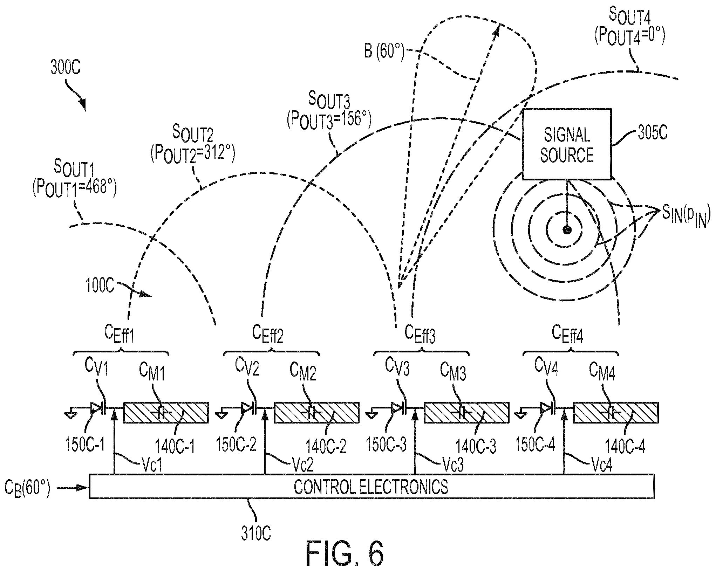

FIG. 6 is a cross-sectional side view showing a simplified metamaterial-based phased array system 300C for generating an emitted radio frequency energy beam B in accordance with another embodiment. Phased array system 300C generally includes a signal source 305C, a phase shifting element array 100C, and a control circuit 310C. Signal source 305C is constructed and operates in the manner described above with reference to apparatus 200A to generate an input signal S_{IN} having a specified radio wave frequency and an associated input phase p_{IN}.

According to some aspects, phase shifting element array 100C includes multiple (in this case four) metamaterial structures 140C-1 to 140C-4 that are disposed in a predetermined coordinated pattern, where each of the metamaterial structures is configured in the manner described above to resonate at the radio wave frequency of input signal S_{IN} in order to respectively produce output signals S_{OUT1} to S_{OUT4}. For example, metamaterial structure 140C-1 fixed capacitance C_{M1} and is otherwise configured to resonate at the radio wave frequency of input signal S_{IN} in order to produce output signal S_{OUT1}. Similarly, metamaterial structure 140C-2 has fixed capacitance C_{M2}, metamaterial structure 140C-3 has fixed capacitance C_{M3}, and metamaterial structure 140C-4 has fixed capacitance C_{M4}, where metamaterial structures 140C-2 to 140C-4 are also otherwise configured to resonate at the radio wave frequency of input signal S_{IN} to produce output signals S_{OUT2}, S_{OUT3} and S_{OUT4}, respectively. The coordinated pattern formed by metamaterial structures 140C-1 to 140C-4 is selected such that output signals S_{OUT1} to S_{OUT4} combine to produce an electro-magnetic wave. Although four metamaterial structures are utilized in the exemplary embodiment, this number is arbitrarily selected for illustrative purposes and brevity, and array 100C may be produced with any number of metamaterial structures.

Similar to the single element embodiments described above, phase shifting element array 100C also includes variable capacitors 150C-1 to 150C-4 that are coupled to associated metamaterial structures 140C-1 to 140C-4 such that effective capacitances C_{eff1} to C_{eff4} of metamaterial structures 140C-1 to 140C-4 are respectively altered corresponding changes in variable capacitances C_{V1} to C_{V4}, which in turn are generated in accordance with associated applied phase control voltages Vc1 to Vc4. For example, variable capacitor 150C-1 is coupled to metamaterial structure 140C-1 such that effective capacitance C_{eff1} is altered by changes in variable capacitance C_{V1}, which in turn changes in accordance with applied phase control voltage Vc1.

According to another aspect of the present embodiment, control circuit 310C is configured to independently control the respective output phases p_{OUT1} to p_{OUT4} of output signals S_{OUT1} to S_{OUT4} using a predetermined set of variable capacitances C_{V1} to C_{V4} that are respectively applied to metamaterial structures 140C-1 to 140C-4 such that output signals S_{OUT1} to S_{OUT4} cumulatively generate emitted beam B in a desired direction. That is, as understood by those skilled in the art, by generating output signals S_{OUT1} to S_{OUT4} with a particular coordinated set of output phases p_{OUT1} to p_{OUT4}, the resulting combined electro-magnetic wave produced by phase shifting element array 100C is reinforced in the desired direction and suppressed in undesired directions, thereby producing beam B emitted in the desired direction from the front of array 100C. By predetermining a combination (set) of output phases p_{OUT1} to p_{OUT4} needed to produce beam B in a particular direction, and by predetermining an associated combination of phase control voltages Vc1 to Vc4 needed to produce this combination of output phases p_{OUT1} to p_{OUT4}, and by constructing control circuit 310C such that the associated combination of phase control voltages Vc1 to Vc4 are generated in response to a beam control signal C_{B} having a signal value equal to the desired beam direction, embodiments described herein facilitate the selective generation of radio frequency beam that are directed in a desired direction. For example, as depicted in FIG. 6, in response to a beam control signal C_{B} having a signal value equal to a desired beam direction of 60°, control circuit 310C generates an associated combination of phase control voltages Vc1 to Vc4 that cause metamaterial structures 140C-1 to 140C-4 to generate output signals S_{OUT1} to S_{OUT4} at output phases p_{OUT1} to p_{OUT4} of 468°, 312°, 156° and 0°, respectively, whereby output signals S_{OUT1} to S_{OUT4} cumulatively produce emitted beam B at the desired 60°angle.

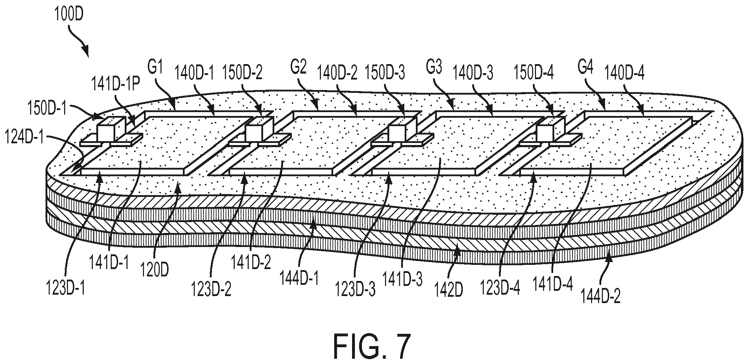

FIG. 7 is a simplified perspective and cross-sectional view showing a phase shifting element array 100D in which metamaterial structures 140D-1 to 140D-4 are formed using the three-layered structure described above with reference to FIGS. 3A and 3B, and arranged in a one-dimensional array and operably coupled to variable capacitors 150D-1 to 150D-4, respectively. Similar to the single element embodiment described above, phase shifting element array 100D includes an electrically isolated (floating) metal backplane layer 142D, and (lossless) dielectric layers 144D-1 and 144D-2 disposed above and below backplane layer 142D.

As indicated in FIG. 7, each metamaterial structure (e.g., structure 140D-1) includes a metal island structure 141D-1 disposed on upper dielectric layer 144D-1 and effectively includes an associated backplane layer portion 142D-1 of backplane layer 142D disposed under metal island structure 141D-1 with an associated portion of the dielectric layer 144A-1 sandwiched therebetween). For example, metamaterial structure 140D-1 includes island structure 141D-1, backplane layer portion 142D-1, and an associated portion of upper dielectric layer 144A-1 that is sandwiched therebetween. Similarly, metamaterial structure 140D-2 includes island structure 141D-2 and backplane layer portion 142D-2, metamaterial structure 140D-3 includes island structure 141D-3 and backplane layer portion 142D-3, and metamaterial structure 140D-4 includes island structure 141D-4 and backplane layer portion 142D-4. Consistent with the single element description provided above, each associated metal island structure and backplane layer portion are cooperatively configured (e.g., sized and spaced) such that each metamaterial structure resonates at a specified radio frequency. For example, metal island structure 141D-1 and backplane layer portion 142D-1 are cooperatively configured to produce a fixed capacitance that causes metamaterial structure 140D-1 to resonate at a specified radio frequency.

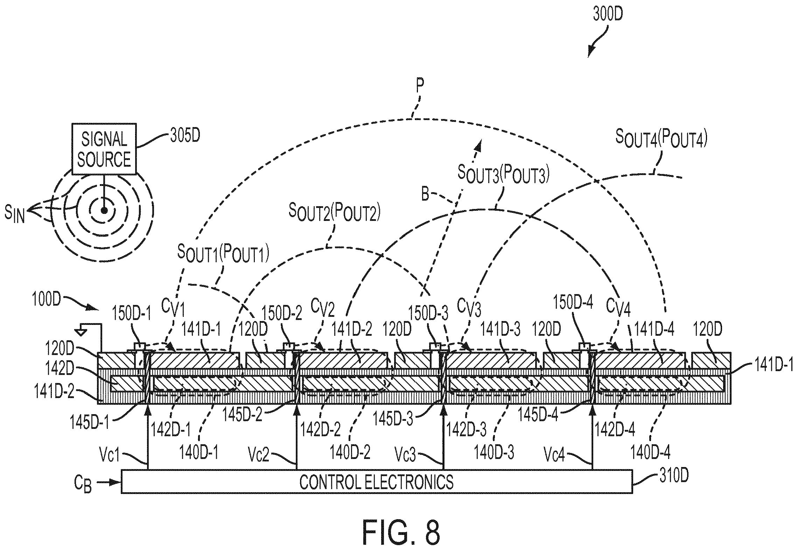

As indicated in FIG. 8, phase shifting element array 100D further includes a base metal structure 120D disposed on upper dielectric layer 141D-1 that is spaced (e.g., electrically isolated) from each of metal island structures 141D-1 to 141D-4 in a manner similar to the single element embodiment described above. In this case, base metal structure 120D defines four openings 123D-1 to 123D-4, each having an associated inner peripheral edge that is separated from an outer peripheral edge of associated metal island structures 141D-1 to 141D-4 by way of peripheral gaps G1 to G4 (e.g., island structures 141D-1 is disposed in opening 123D-1 and is separated from base metal structure 120D by gap G1). Variable capacitors 150D-1 to 150D-4 respectively extend across gaps G1 to G4, and have one terminal connected to an associated metal island structure 141D-1 to 141D-4, and a second terminal connected to base metal structure 120D (e.g., variable capacitor 150D-1 extends across gap G1 between metal island structure 141D-1 and base metal structure 120D). Base metal structure 120D and metal island structures 141D-1 to 141D-4 are preferably formed by etching a single metal layer (e.g., both comprise the same metal composition, e.g., copper).

FIG. 8 also shows phase shifting element array 100D incorporated into a phased array system 300D that includes a signal source 305D and a control circuit 310D. Signal source 305D is configured to operate in the manner described above to generate input signal S_{IN} having the resonance radio frequency of metamaterial structures 140D-1 to 140D-4. Control circuit 310D is configured to generate phase control voltages Vc1 to Vc4 that are transmitted to variable capacitors 150D-1 to 150D-4, respectively, by way of metal via structures 145D-1 to 145D-4 in the manner described above, whereby variable capacitors 150D-1 to 150D-4 are controlled to apply associated variable capacitances C_{V1} to C_{V4} onto metal island structures 141D-1 to 141D-4, respectively. According to an aspect of the present embodiment, because metamaterial structures 140D-1 to 140D-4 are aligned in a one-dimensional array (e.g., in a straight line), variations in output phases p_{OUT1} to p_{OUT4} cause resulting beam B to change direction in a planar region (e.g., in the phase shaped, two-dimensional plane P, which is shown in FIG. 8).

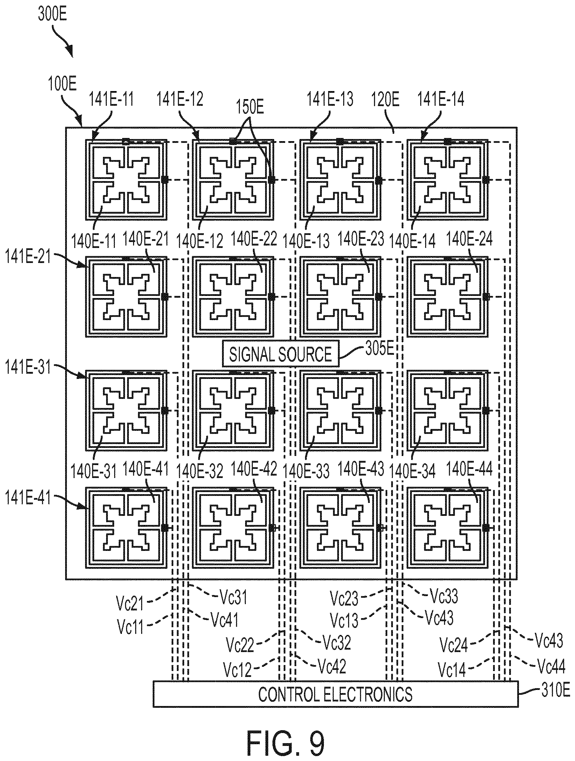

FIG. 9 is simplified top view showing a phased array system 300E including a phase shifting element array 100E having sixteen metamaterial structures 140E-11 to 140E-44 surrounded by a base metal structure 120E, a centrally located signal source 305E, and a control circuit 310E (which is indicated in block form for illustrative purposes, but is otherwise disposed below metamaterial structures 140E-11 to 140E-44).

According to an aspect of the present embodiment, metamaterial structures 140E-11 to 140E-44 are disposed in a two-dimensional pattern of rows and columns, and each metamaterial structure 140E-11 to 140E-44 is individually controllable by way of control voltages V_{C11} to V_{C44}, which are generated by control circuit 310E and transmitted by way of conductive structures (depicted by dashed lines) in a manner similar to that described above. Specifically, uppermost metamaterial structures 140E-11, 140E-12, 140E-13 and 140E-14 form an upper row, with metamaterial structures 140E-21 to 140E-24 forming a second row, metamaterial structures 140E-31 to 140E-34 forming a third row, and metamaterial structures 140E-41 to 140E-44 forming a lower row. Similarly, leftmost metamaterial structures 140E-11, 140E-21, 140E-31 and 140E-41 form a leftmost column controlled by control voltages V_{C11}, V_{C21}, V_{C31} and V_{C41}, respectively, with metamaterial structures 140E-12 to 140E-42 forming a second column controlled by control voltages V_{C12} to V_{C42}, metamaterial structures 140E-13 to 140E-43 forming a third column controlled by control voltages V_{C13} to V_{C43}, and metamaterial structures 140E-14 to 140E-44 forming a fourth (rightmost) column controlled by control voltages V_{C14} to V_{C44}.

According to an aspect of the present embodiment, two variable capacitors 150E are connected between each metamaterial structure 140E-11 to 140E-44 and base metal structure 120E. The configuration and purpose of variable capacitors 150E is the same as that provided above, where utilizing two variable capacitors increases the range of variable capacitance applied to each metamaterial structure. In the illustrated embodiment, a single control voltage is supplied to both variable capacitors of each metamaterial structure. In addition, a larger number of variable capacitors may be used.

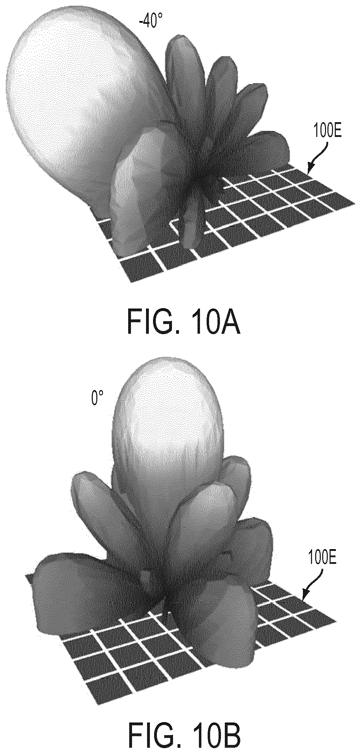

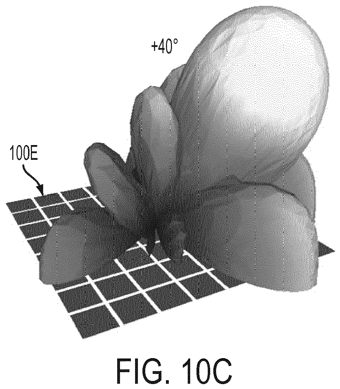

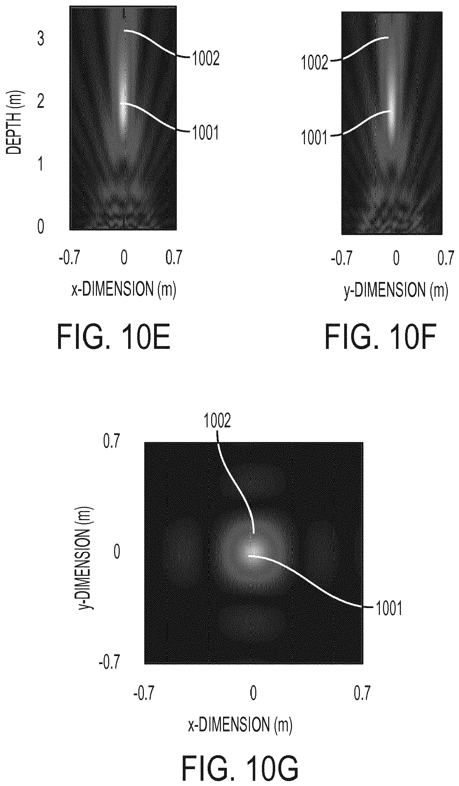

Control circuit 310E is configured to generate phase control voltages V_{c11} to V_{c44} that are transmitted to variable capacitors 150E of each metamaterial structure 140E-11 to 140E-44, respectively, such that variable capacitors 150E are controlled to apply associated variable capacitances to generate associated output signals having individually controlled output phases. According to an aspect of the present embodiment, because metamaterial structures 140E-11 to 140E-44 are arranged in a two-dimensional array (e.g., in rows and columns), variations in output phases cause resulting beams to change direction in an area defined by a three-dimensional region, shown in FIGS. 10A to 10C. Specifically, FIGS. 10A, 10B and 10C are diagrams depicting the radiation pattern at 0, +40 and -40 degrees beam steer. The radiation pattern consists of a main lobe and side lobes. The side lobes represent unwanted radiation in undesired directions.

The system including the metamaterial phased array described herein can be configured to provide localized heat to a tumor site non-invasively. The output signal at the tumor site (e.g., the target region) can be dynamically controlled with respect to depth control, steering, shape, focus and intensity to provide a predetermined therapeutic heat flux for treating the tumor. Destructive interference of the component output signals of the phased array elements nullifies the EM field at the surface of the body and other non-targeted areas, thus the temperature increase in these regions can be controlled to be negligible.

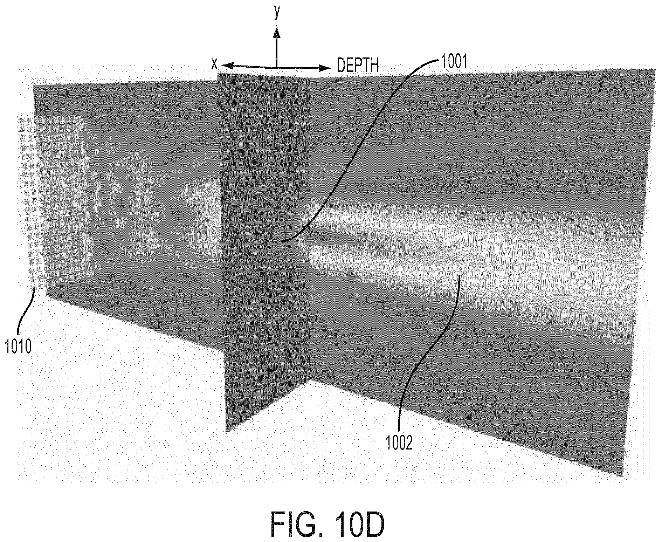

FIGS. 10D-10G show results of spatial modeling the energy of the beamformed output signal from the system. FIGS 10D - 10G demonstrate focusing the local hyperthermia beam in x, y, and depth (into the body) dimensions using the metamaterials phased array 1010 as described herein, where darker shaded regions 1001 of the beam indicate regions of higher energy of the output signal and lighter shaded regions 1002 of the beam indicate lower energy regions of the output signal.

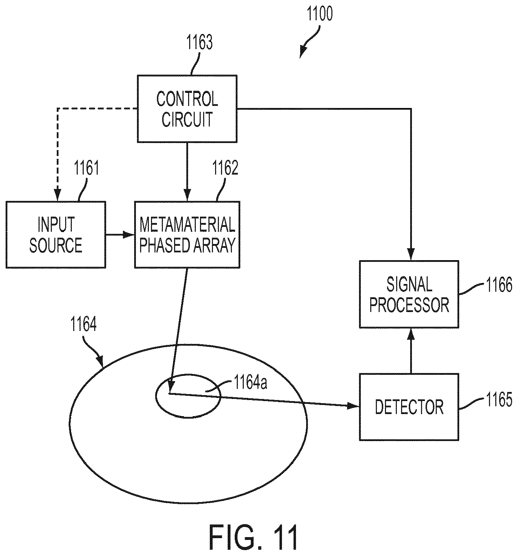

Some embodiments involve an imaging system as illustrated in the simplified block diagram of FIG. 11. The illustrated system1 100 includes a control system 1163 configured to control the phases and/or amplitudes of the respective component output signals of the elements of the phased array 162 resonating at a frequency of an input signal from an input source. The phases and/or amplitudes of the phased array elements are controlled so that the output signal is caused to scan or "sweep" an area 1164a of a patient's body 1164 into which the output signal is directed. The control circuit 1163 generates scan direction data that includes the instantaneous scan direction of the output signal. The system includes a receiver circuit 1165 configured to detect a portion of the output signal that is reflected from one or more structures in the scan area 164a interior to the body. A signal processing circuit 1166 combines information from the scan direction and the detected portion of the output signal to provide information related to the presence and location of the structures.

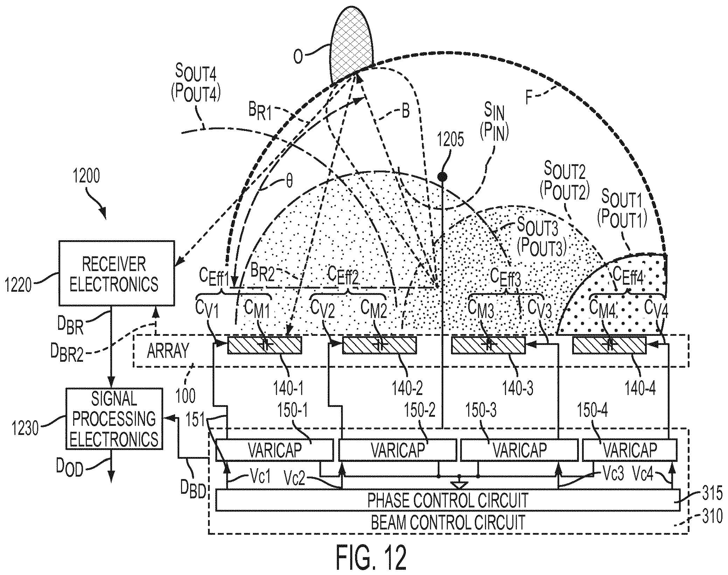

FIG. 12 is a simplified side view diagram showing an imaging system 1200 according to an exemplary embodiment. System 1200 generally includes a signal source 1205, a phase shifting element array 100 and a beam control circuit 1210 for generating a scan beam B that is directed into a target region F. System 1200 also includes a receiver circuit 1220 for processing reflected beam portions B_{R} from target region F, and a signal processing circuit 1230 for processing reflected beam data to determine presence, position, and/or image information related to a body structure O that might be present in area F.

Signal source 1205 is a signal transmission source (e.g., a feed horn or a leaky-wave feed) disposed in close proximity to phase shifting element array 100, and is configured to generate a radio wave frequency input signal S_{IN} at a particular radio wave frequency (e.g., in the range of 3 kHz to 300 GHz) and an input phase p_{IN}. As previously discussed, the radio wave frequency of input signal S_{IN} is generated to match resonance characteristics of phase shifting element array 100.

According to the exemplary embodiment, phase shifting element array 100 includes four metamaterial structures 140-1 to 140-4, each configured to resonate at the radio wave frequency of input signal S_{IN} such that phase shifting element array 100 generates four output signals S_{OUT1} to S_{OUT4}, each having the radio wave frequency and an associated output phase p_{OUT1} to p_{OUT4}. For example, when metamaterial structure 140-1 is configured to resonate at 2.4GHz and input signal S_{IN} is generated at 2.4GHz, metamaterial structure 140-1 generates output signal S_{OUT1} at 2.4GHz by retransmitting (e.g., reflecting/scattering) input signal S_{IN}. When all four metamaterial structures 140-1 to 140-4 are configured in this manner and subjected to input signal S_{IN}, array 100 produces four separate output signals S_{OUT1} to S_{OUT4}, each having a frequency of 2.4GHz. According to a presently preferred embodiment, metamaterial structures 140-1 to 140-4 may be layered metal-dielectric composite architectures, as described with reference to FIGS. 3-5, but may be engineered in a different form, provided the resulting structure is configured to resonate at the radio frequency of applied input signal S_{IN}, and has a large phase swing near resonance. In providing this resonance characteristic, metamaterial structures 140-1 to 140-4 are produced with associated inherent "fixed" capacitances C_{M1} to C_{M4} and associated inductances that collectively provide the desired resonance characteristics. As understood in the art, the term "metamaterial" identifies an artificially engineered structure formed by two or more materials and multiple elements that collectively generate desired electromagnetic properties, where metamaterial achieves the desired properties not from its composition, but from the exactingly-designed configuration (e.g., the precise shape, geometry, size, orientation and arrangement) of the structural elements formed by the materials. As used herein, the phrase "metamaterial structure" is intended to mean a dynamically reconfigurable/tunable metamaterial and large phase swing properties suitable for the purpose set forth herein. Metamaterial structures achieve their desired effects by incorporating structural elements of sub-wavelength sizes, e.g. features that are actually smaller than the radio frequency wavelength of the waves they affect. Although four metamaterial structures are utilized in the exemplary embodiment, this number is arbitrarily selected for illustrative purposes and brevity, and array 100 may be alternatively produced with any number of metamaterial structures.

According to another aspect, beam control circuit 1230 comprises integrated circuitry configured to generate and apply four variable capacitances C_{V1} to C_{V4} onto metamaterial structures 140-1 to 140-4, respectively, such that an effective capacitance of each metamaterial structure is altered by a corresponding change in the applied variable capacitance C_{V1}. As mentioned above, each metamaterial structure 140-1 to 140-4 is produced with associated inherent "fixed" (unchanging) capacitances C_{M1} to C_{M4}, respectively. The effective capacitance of each metamaterial structure 140-1 to 140-4 is generated by a product of the structure's inherent (fixed) capacitance and an associated applied variable capacitance. For example, metamaterial structure 140-1 has an effective (operating) capacitance C_{eff1} generated by inherent (fixed) capacitance C_{M1} and associated variable capacitance C_{V1}, which is applied onto metamaterial structure 140-1 by beam control circuit 130 using techniques described below. Similarly, metamaterial structure 140-2 has an effective (operating) capacitance C_{eff2} generated by inherent capacitance C_{M2} and associated applied variable capacitance C_{V2}, metamaterial structure 140-3 has an effective (operating) capacitance C_{eff3} generated by inherent capacitance C_{M3} and associated applied variable capacitance C_{V3}, and metamaterial structure 140-4 has an effective (operating) capacitance C_{eff4} generated by inherent capacitance C_{M4} and associated applied variable capacitance C_{V4}. Some embodiments achieve control over output phase p_{OUT1} to p_{OUT4} of radio frequency (output) signals S_{OUT1} to S_{OUT4} without the use of conventional phase shifters simply by controlling variable capacitances C_{V1} to C_{V4} applied to metamaterial structures 140-1 to 140-4.

According to another aspect, beam control circuit 310 is further configured to coordinate and vary (e.g., change over time) variable capacitances C_{V1} to C_{V4} applied to metamaterial structures 140-1 to 140-4 such that beam B (generated by way of output signals S_{OUT1} to S_{OUT4}, collectively) scans, or "sweeps", across target region F at a predetermined rate over a predetermined scan range (pattern). That is, at each instant a particular set of variable capacitances C_{V1} to C_{V4} are applied to metamaterial structures 140-1 to 140-4 such that output signals S_{OUT1} to S_{OUT4} have correspondingly different output phases p_{OUT1} to p_{OUT4} (e.g., output signal S_{OUT1} is generated at output phase p_{OUT1} that is different from output phase p_{OUT2} of output signal S_{OUT2}), and output phases p_{OUT1} to p_{OUT4} are coordinated such that output signals S_{OUT1} to S_{OUT4} cumulatively emit scan beam B in a direction determined by the instantaneous set of output phase values. At the beginning of each scan pass, the output phases p_{OUT1} to p_{OUT4} are coordinated such that beam B is directed along an initial direction (e.g., a scan angle of - 60°, corresponding to a leftmost beam angle). As understood in the art, by coordinating output phases p_{OUT1} to p_{OUT4} in this way, the combined electro-magnetic wave generated by output signals S_{OUT1} to S_{OUT4} is reinforced in a particular "desired" direction, and suppressed in undesired directions, whereby the scan beam B is emitted at a desired angle from the front of array 100. Output phases p_{OUT1} to p_{OUT4} are subsequently varied such that beam B begins sweeping from the initial direction toward a central direction (e.g., directly in front of array 100, and corresponding to a scan angle of 0°), and then continues to sweep from the central direction to an ending direction (e.g., a scan angle of +60°, corresponding to a rightmost beam angle). The scan rate and repeat/refresh rate at which beam B is generated is determined by the rate at which output phases p_{OUT1} to p_{OUT4} are varied. Beam control circuit 1210 generates these output phases p_{OUT1} to p_{OUT4} changes by changing the variable capacitances C_{V1} to C_{V4} applied to metamaterial structures 140-1 to 140-4 over time (e.g., in accordance with predefined time-based functions), whereby beam control circuit 1210 causes beam B to scan (sweep) across target field F in a characteristic "radar-like" sweep pattern.

According to some embodiments, beam control circuit 1210 is implemented using variable capacitors (varicaps) 150-1 to 150-4 and a phase control circuit 1215, where variable capacitors 150-1 to 150-4 are respectively coupled to metamaterial structures 140-1 to 140-4, and are controlled by way of phase control voltages Vc1 to Vc4 generated by phase control circuit 1215 (e.g., variable capacitor 150-1 generates variable capacitance C_{V1} having a capacitance level that is proportional to the voltage level of phase control voltage Vc1). As understood in the art, variable capacitors are typically two-terminal electronic devices configured to produce a capacitance that is intentionally and repeatedly changeable by way of an applied electronic control signal. In this case, variable capacitors 150-1 to 150-4 are coupled to metamaterial structures 140-1 to 140-4 such that respective effective capacitances C_{eff1} to C_{eff4} of metamaterial structures 140-1 to 140-4 are determined by a product of inherent capacitance C_{M1} to C_{M4} and variable capacitances C_{V1} to C_{V4} supplied by variable capacitors 150-1 to 150-4. For example, effective capacitance C_{eff1} of metamaterial structure 140-1 is determined by inherent capacitance C_{M1} and variable capacitance C_{V1}, which is supplied to metamaterial structure 140-1 during operation by variable capacitor 150-1. Because output phase p_{OUT1} is determined in part by effective capacitance C_{eff1}, output signal S_{OUT1} is "tunable" (adjustably controllable) to a desired phase value by way of changing variable capacitance C_{V1}, and this is achieved by way of changing the phase control signal Vc1 applied to variable capacitor 150-1.

Phase control voltages Vc1 to Vc4 are applied across variable capacitors 150-1 to 150-4 such that each variable capacitance C_{V1} to C_{V4} is applied to metamaterial structures 140-1 to 140-4, respectively. For example, variable capacitor 150-1 includes a first terminal 151 connected to metamaterial structure 140-1 and a second terminal 152 connected to ground, whereby variable capacitor 150-1 generates associated variable capacitance C_{V1} having a capacitance level that varies in accordance with the voltage level of phase control voltage Vc1 in the manner illustrated in FIG. 2 (e.g., the capacitance level of variable capacitance C_{V1} changes in direct proportion to phase control voltage Vc1). As indicated in FIG. 1, variable capacitors 150-2 to 150-4 are similarly connected, and share a common voltage source (e.g., ground) with variable capacitor 150-1. In an alternative embodiment, the conductive structures that transmit phase control voltages Vc1 to Vc4 from phase control circuit 1215 are either connected to metamaterial structures 140-1 to 140-4, which in turn are connected to associated variable capacitors 150-1 to 150-4.

Imaging system 1200 further includes a receiver 1220 and signal processing circuitry 1230 that are utilized to detecting body structures in target region F, and to generate target location data that can be utilized, for example. In the exemplary embodiment, phase control circuit 1215 is configured to generate beam direction data D_{BD} indicating an instantaneous beam direction θ of said scan beam B as it sweeps the target region F, and receiver circuitry 1220 is configured to detect portions B_{R} of the scan beam B that are reflected from body structures, e.g., tumors disposed in the target region F. Receiver circuitry 1220 also generates beam detection data D_{BR} indicating each time a reflected beam portion B_{R} is detected. Signal processing circuitry 1230 is configured to determine the position of each structure O in target region F by correlating beam detection data D_{BR} received from receiver circuitry 1220 with beam direction data D_{BD} received from beam control circuit 310 at the time of beam portion detection. For example, assume the body structure of interest O is disposed a position corresponding to a -45° direction angle relative to array 100. In this case, as beam B sweeps across field F and passes the -45° direction angle, receiver circuitry 1220 generates beam detection data D_{BR} indicating the reception of reflected beam portion B_{R} caused by the presence of structure O, and then signal processing circuitry 1230 correlates the reception of this reflected beam portion B_{R} with beam direction data D_{BD} (e.g., indicating that beam B was directed at -45° when the reflected beam portion was received) to determine the position of structure O. Signal processing circuitry 1230 also optional circuitry for generating other useful information (e.g., providing an image of the body structure, determining the size of the body structure O and/or position of the body structure O with respect to the array 100 or other reference points using known signal processing techniques.

Embodiments described herein involve a therapy and/or imaging system that uses a phase shifting array including a plurality of metamaterial structures that resonate in response to an input electromagnetic (EM) signal. The phase shifting array generates an output EM signal that is a sum of component output electromagnetic signals generated respectively by the metamaterial structures and is configured to propagate wirelessly through at least a portion of a patient's body. A control circuit controls one or both of phases and amplitudes of the component electromagnetic output signals so that at least one of constructive and destructive interference between the component output electromagnetic signals causes the output signal to have a higher intensity EM radiation at a target region interior to the body and to have a zero or low intensity radiation at a non-target region interior to the body. The control circuit is configured to control at least one of position, focus, and intensity of the higher intensity EM radiation at the target region. The higher intensity EM radiation is capable of generating a heat flux suitable to provide hyperthermia therapy at the target region. For example, the EM radiation in the target region may provide a tissue temperature in a range of about 40C to about 60C.

The control circuit includes variable capacitors electrically coupled respectively to the metamaterial structures so that a change in capacitance of one of the variable capacitors changes a phase of a component output signal of an associated metamaterial structure. A control signal generator provides control signals that control capacitances of the variable capacitors. The EM signal propagates wirelessly to the array of metamaterial structures through a wire probe antenna, e.g., through a wire probe antenna and/or a waveguide. Each metamaterial structure include a first metal layer structure, an electrically isolated second metal layer structure, and a dielectric layer disposed between the first and second metal structures. The first and second metal layer structures are cooperatively configured such that the metamaterial structure resonates at a frequency of the input EM signal at a fixed capacitance. In some embodiments, the first metal layer structure is disposed on an upper dielectric surface of the dielectric layer, and a third metal layer structure is disposed on the upper dielectric surface and spaced apart from the first metal layer structure. A variable capacitor of the control circuit has a first terminal electrically coupled to the first metal layer structure and a second terminal electrically coupled to the third metal layer structure.

In some embodiments, system involves a body imaging system wherein the control signal is configured to control the component EM output signals to scan the output signal across the target region. In this embodiment, the control circuit can also be configured to generate beam direction data indicating instantaneous scan direction of the output signal. The imaging system includes a receiver circuit configured to detect a portion of the output signal reflected from structures interior to the body. A signal processing circuit processes the scan direction and the reflected portion of the output signal and to provide information about the structures, wherein the information may include location and/or image information.

As used herein, directional terms such as "upper", "upward", "uppermost", "lower", "lowermost", "front", "rightmost" and "leftmost", are intended to provide relative positions for purposes of description, and are not intended to designate an absolute frame of reference. In addition, the phrases "integrally formed" and "integrally connected" are used herein to describe the connective relationship between two portions of a single fabricated or machined structure, and are distinguished from the terms "connected" or "coupled" (without the modifier "integrally"), which indicates two separate structures that are joined by way of, for example, adhesive, fastener, clip, or movable joint.

The foregoing description of various embodiments has been presented for the purposes of illustration and description and not limitation. The embodiments disclosed are not intended to be exhaustive or to limit the possible implementations to the embodiments disclosed. Many modifications and variations are possible in light of the above teaching without departing from the scope of the invention as defined by the appended claims.

## Claims

1. A device comprising:
a phase shifting element array including a plurality of metamaterial structures that resonate in response to an input electromagnetic (EM) signal, the phase shifting element array generating an output EM signal that is a sum of component output electromagnetic signals generated respectively by the metamaterial structures and is configured to propagate wirelessly through at least a portion of a patient's body ;
a control circuit configured to control one or both of phases and amplitudes of the component electromagnetic output signals so that at least one of constructive and destructive interference between the component output electromagnetic signals causes the output signal to have a higher intensity EM radiation at a target region interior to the body and to have a zero or low intensity radiation at a non-target region interior to the body, and so that the output signal is caused to scan an area of a patient's body into which the output signal is directed, and is configured to generate scan direction data that includes the instantaneous scan direction of the output signal;
a receiver circuit configured to detect a portion of the output signal that is reflected from one or more structures in the scan area interior to the body; and
a signal processing circuit configured to combine information from the scan direction and the detected portion of the output signal to provide image information related to a body structure that might be present in the area.

2. The device of claim 1, wherein the higher intensity electromagnetic radiation generates a tissue temperature suitable for hyperthermia therapy at the target region.

3. The device of claim 2, wherein the tissue temperature at the target region is in range of about 40 C to 50 C.

4. The device of claim 2, wherein the control circuit is configured to control at least one of position, focus, and intensity of the higher intensity EM radiation at the target region.

5. The device of claim 1, wherein the control circuit comprises:
variable capacitors electrically coupled respectively to the metamaterial structures so that a change in capacitance of one of the variable capacitors changes a phase of a component output signal of an associated metamaterial structure; and
a control signal generator configured to generate control signals that control capacitances of the variable capacitors.

6. The device of claim 1, wherein the input EM signal is propagated wirelessly to the array of metamaterial structures through a wire probe antenna.

7. The device of claim 1, wherein each metamaterial structure comprises:
a first metal layer structure;
an electrically isolated second metal layer structure; and
a dielectric layer disposed between the first and second metal structures, wherein the first and second metal layer structures are cooperatively configured such that the metamaterial structure resonates at a frequency of the input EM signal at a fixed capacitance.

8. The device of claim 7, wherein:
the first metal layer structure is disposed on an upper dielectric surface of the dielectric layer;
the metamaterial structure further comprises a third metal layer structure disposed on the upper dielectric surface and spaced apart from the first metal layer structure; and
a variable capacitor has a first terminal electrically coupled to the first metal layer structure and a second terminal electrically coupled to the third metal layer structure.

9. The device of claim 7, wherein:
the first metal layer structure is disposed on an upper dielectric surface of the dielectric layer;
the metamaterial structure further comprises a third metal layer structure disposed on the upper dielectric surface and spaced apart from the first metal layer structure;
a second metamaterial structure further comprises a fourth metal layer structure disposed on the lower dielectric surface and spaced apart from the second metal layer structure; and
a variable capacitor has a first terminal electrically coupled to the second metal layer structure and a second terminal electrically coupled to the fourth metal layer structure.

10. A method comprising:
generating an output EM signal that is a sum of component output electromagnetic signals generated respectively by a plurality of metamaterial structures that resonate in response to an input electromagnetic (EM) signal;
propagating the output EM wirelessly through at least a portion of a patient's body; and
controlling one or both of phases and amplitudes of the component electromagnetic output signals so that at least one of constructive and destructive interference between the component output electromagnetic signals causes the output signal to scan an area of a patient's body into which the output signal is directed, generating scan direction data that includes the instantaneous scan direction of the output signal;
detecting a portion of the output signal that is reflected from one or more structures in the scan area interior to the body;
combining information from the scan direction and the detected portion of the output signal to provide image information related to a body structure that might be present in the area.

## Patentansprüche

1. Einrichtung mit:
einem Phasenschieber-Elementarray mit mehreren Meta-Materialstrukturen, die in Reaktion auf ein elektromagnetisches (EM) Eingangssignal in Resonanz schwingen, wobei das Phasenschieber-Elementarray ein EM Ausgangssignal erzeugt, das eine Summe aus elektromagnetischen Komponentenausgangssignalen ist, die entsprechend von den Meta-Materialstrukturen erzeugt werden und die geeignet sind, sich durch zumindest einen Teil eines Patientenkörpers drahtlos auszubreiten;
einer Steuerschaltung, die ausgebildet ist, Phasen und/oder Amplituden der elektromagnetischen Komponentenausgangssignale so zu steuern, dass konstruktive und/oder destruktive Interferenz zwischen den elektromagnetischen Komponentenausgangssignalen bewirken, dass das Ausgangssignal eine höhere EM Strahlungsintensität in einem Zielgebiet im Inneren des Körpers und eine Strahlungsintensität, die Null oder niedrig ist, in einem Nicht-Ziel-Gebiet im Inneren des Körpers hat, und dass bewirkt wird, dass das Ausgangssignal einen Bereich eines Patientenkörpers abtastet, in den das Ausgangssignal gelenkt wird, und ausgebildet ist, Abtastrichtungsdaten zu erzeugen, die die momentane Abtastrichtung des Ausgangssignals enthalten;
einer Empfängerschaltung, die ausgebildet ist, einen Teil des Ausgangssignals, der aus einer oder mehreren Strukturen in dem Abtastbereich im Inneren des Körpers reflektiert wird, zu erfassen; und
einer Signalverarbeitungsschaltung, die ausgebildet ist, Information aus der Abtastrichtung und dem erfassten Teil des Ausgangssignals zu kombinieren, so dass eine Bildinformation, die einen Körperaufbau betrifft, der potentiell in dem Bereich vorhanden ist, bereitgestellt wird.

2. Einrichtung nach Anspruch 1, wobei die elektromagnetische Strahlung mit höherer Intensität eine Gewebetemperatur hervorruft, die für eine Hyperthermie-Therapie in dem Zielgebiet geeignet ist.

3. Einrichtung nach Anspruch 2, wobei die Gewebetemperatur in dem Zielgebiet im Bereich von ungefähr 40° C bis 50° C liegt.

4. Einrichtung nach Anspruch 2, wobei die Steuerschaltung ausgebildet ist, Position und/oder Fokus und/oder Intensität der EM Strahlung mit höherer Intensität in dem Zielgebiet zu steuern.

5. Einrichtung nach Anspruch 1, wobei die Steuerschaltung umfasst:
variable Kondensatoren, die entsprechend mit Meta-Materialstrukturen so elektrisch gekoppelt sind, dass eine Änderung der Kapazität eines der variablen Kondensatoren eine Phase eines Komponentenausgangssignals einer zugehörigen Meta-Materialstruktur ändert; und
einen Steuersignalgenerator, der ausgebildet ist, Steuersignale zu erzeugen, die Kapazitäten der variablen Kondensatoren steuern.

6. Einrichtung nach Anspruch 1, wobei sich das EM Signal drahtlos zu dem Array aus Meta-Materialstrukturen über eine Drahtsondenantenne ausbreitet.

7. Einrichtung nach Anspruch 1, wobei jede Meta-Materialstruktur aufweist:
eine erste Metallschichtstruktur;
eine elektrisch isolierte zweite Metallschichtstruktur; und
eine dielektrische Schicht, die zwischen der ersten und der zweiten Metallschichtstruktur angeordnet ist, wobei die erste und die zweite Metallschichtstruktur im Zusammenwirken derart ausgebildet sind, dass die Meta-Materialstruktur mit einer Frequenz des EM Eingangssignals bei festgelegter Kapazität in Resonanz schwingt.

8. Einrichtung nach Anspruch 7, wobei:
die erste Metallschichtstruktur auf einer oberen dielektrischen Oberfläche der dielektrischen Schicht angeordnet ist;
die Meta-Materialstruktur ferner eine dritte Metallschichtstruktur aufweist, die auf der oberen dielektrischen Oberfläche angeordnet und von der ersten Metallschichtstruktur beabstandet ist; und
ein variabler Kondensator einen ersten Anschluss, der elektrisch mit der ersten Metallschichtstruktur verbunden ist, und einen zweiten Anschluss hat, der elektrisch mit der dritten Metallschichtstruktur verbunden ist.

9. Einrichtung nach Anspruch 7, wobei:
die erste Metallschichtstruktur auf einer oberen dielektrischen Oberfläche der dielektrischen Schicht angeordnet ist;
die Meta-Materialstruktur ferner eine dritte Metallschichtstruktur aufweist, die auf der oberen dielektrischen Oberfläche angeordnet und von der ersten Metallschichtstruktur beabstandet ist;
eine zweite Meta-Materialstruktur ferner eine vierte Metallschichtstruktur aufweist, die auf der unteren dielektrischen Oberfläche angeordnet und von der zweiten Metallschichtstruktur beabstandet ist; und
ein variabler Kondensator einen ersten Anschluss, der elektrisch mit der zweiten Metallschichtstruktur verbunden ist, und einen zweiten Anschluss hat, der elektrisch mit der vierten Metallschichtstruktur verbunden ist.

10. Verfahren mit:
Erzeugen eines elektromagnetischen (EM) Ausgangssignals, das eine Summe von elektromagnetischen Komponentenausgangssignalen ist, die entsprechend von mehreren Meta-Materialstrukturen erzeugt werden, die in Reaktion auf ein elektromagnetisches (EM) Eingangssignal in Resonanz schwingen;
zur Ausbreitung bringen des EM Ausgangssignals in drahtloser Weise durch zumindest einen Teil eines Patientenkörpers; und
Steuern von Phasen und/oder Amplituden der elektromagnetischen KomponentenAusgangssignale derart, dass konstruktive und/oder destruktive Interferenz zwischen den elektromagnetischen Komponentenausgangssignalen bewirken, dass das Ausgangssignal einen Bereich eines Patientenkörpers abtastet, in den das Ausgangssignal gelenkt wird, wodurch Abtastrichtungsdaten erzeugt werden, die die momentane Abtastrichtung des Ausgangssignals enthalten;
Erfassen eines Teils des Ausgangssignals, der von einer oder mehreren Strukturen in dem Abtastbereich im Inneren des Körpers reflektiert wird;
Kombinieren von Information aus der Abtastrichtung und dem erfassten Bereich des Ausgangssignals derart, dass Bildinformation, die einen Körperaufbau betrifft, der potentiell in dem Bereich vorhanden ist, bereitgestellt wird.

## Revendications

1. Dispositif comprenant :
un réseau d'éléments déphaseurs comprenant une pluralité de structures à métamatériaux qui résonnent en réponse à un signal électromagnétique (EM) d'entrée, le réseau d'éléments déphaseurs générant un signal EM de sortie qui est une somme des signaux de sortie électromagnétiques des composants générés respectivement par les structures à métamatériaux et est conçu pour se propager sans fil dans un moins une partie du corps du patient ;
un circuit de commande conçu pour commander l'une ou l'autre ou à la fois les phases et amplitudes des signaux de sortie électromagnétiques des composants de façon qu'au moins une interférence constructive et/ou destructive entre les signaux de sortie électromagnétiques des composants amène le signal de sortie à avoir un rayonnement EM d'intensité élevée dans une région cible à l'intérieur du corps et à avoir un rayonnement d'intensité nulle ou basse dans une région non-cible à l'intérieur du corps, et de façon que le signal de sortie soit amené à balayer une zone du corps du patient sur laquelle le signal de sortie est dirigé, et est conçu pour générer des données de sens de balayage qui comprennent le sens de balayage instantané du signal de sortie ;
un circuit récepteur conçu pour détecter la partie du signal de sortie qui est réfléchie par une ou plusieurs structures dans la zone de balayage à l'intérieur du corps ; et
un circuit de traitement de signal conçu pour combiner l'information relative au sens de balayage et la partie détectée du signal de sortie pour générer une information d'image liée à une structure corporelle susceptible d'être présente dans la zone.

2. Dispositif selon la revendication 1, dans lequel le rayonnement électromagnétique d'intensité élevée génère une température tissulaire qui se prête à un traitement hyperthermique dans la région cible.

3. Dispositif selon la revendication 2, dans lequel la température tissulaire dans la région cible est dans la plage d'environ 40 à 50 °C.

4. Dispositif selon la revendication 2, dans lequel le circuit de commande est conçu pour commander au moins la position et/ou la focalisation et/ou l'intensité du rayonnement EM d'intensité élevée dans la région cible.

5. Dispositif selon la revendication 1, dans lequel le circuit de commande comprend :
des condensateurs variables respectivement électriquement couplés aux structures à métamatériaux de façon qu'une variation de capacité de l'un des condensateurs variables modifie la phase d'un signal de sortie de composant d'une structure à métamatériau associée ; et
un générateur de signaux de commande conçu pour générer des signaux de commande qui commandent les capacités des condensateurs variables.

6. Dispositif selon la revendication 1, dans lequel le signal EM d'entrée se propage sans fil jusqu'au réseau de structures à métamatériaux par l'intermédiaire d'une antenne à sonde filaire.

7. Dispositif selon la revendication 1, dans lequel chaque structure à métamatériau comprend :
une première structure de couche métallique ;
une seconde structure de couche métallique électriquement isolée ; et
une couche diélectrique entre les première et seconde structures métalliques, dans lequel les première et seconde structures de couches métalliques sont conçues de manière à coopérer pour que la structure à métamatériau résonne à une fréquence du signal EM d'entrée à une capacité fixée.

8. Dispositif selon la revendication 7, dans lequel :
la première structure de couche métallique est disposée sur une surface diélectrique supérieure de la couche diélectrique ;
la structure à métamatériau comprend en outre une troisième structure de couche métallique disposée sur la surface diélectrique supérieure et séparée de la première structure de couche métallique ; et
un condensateur variable a une première borne électriquement couplée à la première structure de couche métallique et une seconde borne électriquement couplée à la troisième structure de couche métallique.

9. Dispositif selon la revendication 7, dans lequel :
la première structure de couche métallique est disposée sur une surface diélectrique supérieure de la couche diélectrique ;
la structure à métamatériau comprend en outre une troisième structure de couche métallique disposée sur la surface diélectrique supérieure et séparée de la première structure de couche métallique ;
une seconde structure à métamatériau comprend en outre une quatrième structure de couche métallique disposée sur la surface diélectrique inférieure et séparée de la deuxième structure de couche métallique ; et
un condensateur variable a une première borne électriquement couplée à la deuxième structure de couche métallique et une seconde borne électriquement couplée à la quatrième structure de couche métallique.

10. Procédé comprenant :
la génération d'un signal EM de sortie qui est une somme des signaux de sortie électromagnétiques des composants générés respectivement par une pluralité de structures à métamatériaux qui résonnent en réponse à un signal électromagnétique (EM) d'entrée ;
la propagation sans fil du signal EM de sortie dans au moins une partie du corps du patient ; et
la commande de l'une ou l'autre ou à la fois des phases et amplitudes des signaux de sortie électromagnétiques des composants de façon qu'au moins une interférence constructive et/ou destructive entre les signaux de sortie électromagnétiques des composants amène le signal de sortie à balayer une zone du corps du patient sur laquelle le signal de sortie est dirigé, et à générer des données de sens de balayage qui comprennent le sens de balayage instantané du signal de sortie ;
la détection de la partie du signal de sortie qui est réfléchie par une ou plusieurs structures dans la zone de balayage à l'intérieur du corps ;
la combinaison de l'information relative au sens de balayage et de la partie détectée du signal de sortie pour générer une information d'image liée à une structure corporelle susceptible d'être présente dans la zone.
